(19) Europäisches Patentamt
European Patent Office
Office européen des brevets

(11) Veröffentlichungsnummer: **0 026 736**
**B1**

(12) # EUROPÄISCHE PATENTSCHRIFT

(45) Veröffentlichungstag der Patentschrift: **05.12.84**

(51) Int. Cl.³: **C 07 D 213/61**, C 07 F 9/40

(21) Anmeldenummer: **80810275.0**

(22) Anmeldetag: **04.09.80**

(54) **Verfahren zur Herstellung von 2,3,5-Trichlorpyridin sowie Ammoniumsalze des Methanphosphonsäuremonomethylesters und deren Herstellung.**

(30) Priorität: **10.09.79 US 74216**

(43) Veröffentlichungstag der Anmeldung: **08.04.81 Patentblatt 81/14**

(45) Bekanntmachung des Hinweises auf die Patenterteilung: **05.12.84 Patentblatt 84/49**

(84) Benannte Vertragsstaaten: **CH DE FR GB LI**

(56) Entgegenhaltungen:
**DE - A - 2 632 136**
**US - A - 4 111 938**

**BULL. SOC. CHIM., Seite 1926 (1965); HOUBEN-WEYL, METHODEN DER ORGANISCHEN CHEMIE, 4. Auflage, Band XII/1, 1963, Georg Thieme Verlag Stuttgart, Seite 411**

(73) Patentinhaber: **CIBA-GEIGY AG, Postfach, CH-4002 Basel (CH)**

(72) Erfinder: **Weis, Claus D., Dr., Bünenmattweg 3, CH-4148 Pfeffingen (CH)**
Erfinder: **Sutter, Peter, Seemättlistrasse 14/2, CH-4132 Muttenz (CH)**

**EP 0 026 736 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von 2,3,5-Trichlorpyridin.

2,3,5-Trichlorpyridin ist ein wertvolles Zwischenprodukt zur Herstellung von herbizid wirksamen α-[4-(3′,5′-Dichlorpyrid-2-yloxy)-phenoxy]-alkancarbonsäuren und deren Derivaten. Die Herstellung und Verwendung solcher
α-[4-(3′,5′-Dichlorpyrid-2-yloxy)-phenoxy]-alkancarbonsäuren
und ihrer Derivate ist beispielsweise in der US-Patentschrift 4 092 151 beschrieben.

Es ist bekannt, 2,3,5-Trichlorpyridin durch Umsetzung von N-Methyl-3,5-dichlor-2-pyridon mit Phosgen herzustellen (Ann. Chem. 486, 71, 78 (1931)). Das als Ausgangsmaterial benötigte N-Methyl-3,5-dichlor-2-pyridon wird durch Chlorierung von N-Methyl-2-pyridon erhalten (J. pr. Chem. 93, 371 (1916)), das seinerseits durch elektrolytische Oxidation von N-Methylpyridiniumsulfat (J. pr. Chem. 93, 363 (1916)) oder durch Umsetzung von 2-Aminopyridin mit Kaliumnitrit in saurem Medium und Methylierung des gebildeten 2-Pyridons mit Dimethylsulfat (Ann. Chem. 489, 109, 110 (1931)) hergestellt werden kann.

Aus der US Patentschrift 4 111 938 ist ferner bekannt, 2,3,5-Trichlorpyridin durch Dechlorierung von Pentachlorpyridin oder 2,3,5,6-Tetrachlorpyridin mit Zinkstaub in alkalischem Medium bei einem pH-Wert über 11 in Gegenwart von Wasser und einem mit Wasser nicht mischbaren Lösungsmittel herzustellen.

Es wurde nun gefunden, dass man 2,3,5-Trichlorpyridin in guter Ausbeute und Reinheit herstellen kann, wenn man 2,3,4,5-Tetrachlorpyridin in einem Alkanphosphonsäuredialkylester (Dialkylalkanphosphonat) mit je 1 bis 4 Kohlenstoffatomen in den Alkylgruppen oder in einem Phosphorsäuretrialkylester (Trialkylphosphat) mit je 1 bis 4 Kohlenstoffatomen in den Alkylgruppen als Lösungsmittel bei 60–120 °C in Gegenwart von 1,4 bis 2,8 Mol pro Mol 2,3,4,5-Tetrachlorpyridin eines Ammoniumsalzes einer anorganischen oder organischen Säure mit 1,2 bis 1,6 Grammatom Zink pro Mol 2,3,4,5-Tetrachlorpyridin umsetzt.

Geeignete Alkanphosphonsäuredialkylester, die erfindungsgemäss als Lösungsmittel verwendet werden können, sind beispielsweise die Dimethyl-, Diäthyl-, Di-n-Propyl-, Diisopropyl-, Di-n-Butyl-, Di-sec.-Butyl-, Diisobutyl- und Di-tert.-Butylester der Methan-, Äthan-, 1-Methyläthan-, 1,1-Dimethyläthan-, Propan-, 1-Methylpropan-, 2-Methylpropan- und Butanphosphonsäure. Bevorzugte Alkanphosphonsäuredialkylester sind Methanphosphonsäuredimethylester und Äthanphosphonsäurediäthylester.

Geeignete Phosphorsäuretrialkylester, die erfindungsgemäss als Lösungsmittel verwendet werden können, sind beispielsweise Phosphorsäuretrimethylester, Phosphorsäuretriäthylester, Phosphorsäuretri-n-propylester, Phosphorsäuretriisopropylester und Phosphorsäuretributylester. Bevorzugte Phosphorsäuretrialkylester sind Phosphorsäuretrimethylester und Phosphorsäuretriäthylester.

Innerhalb des Temperaturbereiches von 60–120 °C, in dem das erfindungsgemässe Verfahren durchgeführt werden kann, sind Temperaturen von 85–90 °C bevorzugt.

Die erfindungsgemäss verwendbaren Ammoniumsalze enthalten als Kation das Ammoniumion oder die davon durch teilweisen oder vollständigen Ersatz der Wasserstoffatome durch Alkyl- und/oder Phenylgruppen abgeleiteten Derivate, wobei die Phenylgruppen durch einfache Substituenten, wie Alkyl, Alkoxy oder Halogen substituiert sein können. Als Anion enthalten die erfindungsgemäss verwendbaren Ammoniumsalze den Rest einer beliebigen anorganischen oder organischen Säure, die in der Lage ist, Ammoniumsalze zu bilden.

Vorteilhaft verwendbare Ammoniumsalze entsprechen der Formel

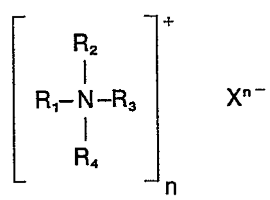
,

in welcher $R_1$, $R_2$, $R_3$ und $R_4$ gleich oder verschieden sein können, und je Wasserstoff, Alkyl mit 1 bis 4 Kohlenstoffatomen oder Phenyl, das gegebenenfalls durch Halogen, Alkylgruppen mit 1 bis 4 Kohlenstoffatomen oder Alkoxygruppen mit 1 bis 4 Kohlenstoffatomen substituiert sein kann, bedeuten, X für ein Anion aus der Gruppe Chlorid, Bromid, Sulfat, Hydrogensulfat, Phosphat, Hydrogenphosphat, Dihydrogenphosphat, Carbonat, Hydrogencarbonat, Acetat, Propionat, Butyrat, Isobutyrat, Oxalat, Benzoat, Alkanphosphonat mit 1 bis 4 Kohlenstoffatomen in der Alkylgruppe und Alkan- bzw. Benzolsulfonat mit 1 bis 4 Kohlenstoffatomen in der Alkylgruppe steht und n 1 bis 3 bedeutet und der Zahl der negativen Ladungen des jeweiligen Anions X entspricht.

Als weitere vorteilhaft verwendbare Ammoniumsalze sind die Ammoniumsalze des Methanphosphonsäuremonomethylesters der Formel

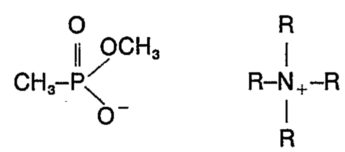
,

in welcher R Wasserstoff oder Methyl bedeutet, zu nennen. Diese Ammoniumsalze stellen neue Verbindungen dar, die in einfacher Weise durch Erhitzen von Ammoniumchlorid oder Tetramethylammoniumchlorid in Methanphosphonsäuredimethylester (Dimethylmethanphosphonat) auf 150 °C unter Abspaltung von Methylchlorid erhalten werden können. Die Umsetzung wird vorteilhaft in überschüssigem Methanphosphonsäuredimethylester als Lösungsmittel durchgeführt. Nach beendigter Umsetzung wird der überschüssige Methanphosphonsäuredimethylester im Vakuum abdestilliert und das Ammoniumsalz, gegebenenfalls nach Digerieren in einem geeigneten Lösungsmittel, z.B. Aceton, Methyläthylketon oder Äther, in kristalliner Form gewonnen.

Bevorzugte Ammoniumsalze sind Ammoniumchlorid, Ammoniumsulfat, Ammoniumcarbonat, das Ammoniumsalz des Methanphosphonsäuremonomethylesters und das Tetramethylammoniumsalz des Methanphosphonsäuremonomethylesters. Besonders bevorzugt sind das Ammoniumsalz des Methanphosphonsäuremonomethylesters und das Tetramethylammoniumsalz des Methanphosphonsäuremonomethylesters.

Die vorgenannten Ammoniumsalze werden bei Verwendung von Methanphosphonsäuredialkylester als Lösungsmittel vorzugsweise in einer Menge von 1,6 Mol pro Mol 2,3,4,5-Tetrachlorpyridin eingesetzt. Bei Verwendung von Äthanphosphonsäuredialkylester oder Phosphorsäuretrialkylester als Lösungsmittel werden vorzugsweise 2,6–2,7 Mol Ammoniumsalz pro Mol 2,3,4,5-Tetrachlorpyridin eingesetzt.

Das erfindungsgemäss zu verwendende Zink wird in Form von Zinkspänen oder vorzugsweise in Form von Zinkstaub eingesetzt. Es werden vorzugsweise 1,20–1,30 Grammatom Zink pro Mol 2,3,4,5-Tetrachlorpyridin verwendet.

Durch das erfindungsgemässe Verfahren wird es möglich, 2,3,5-Trichlorpyridin durch selektive Dechlorierung von 2,3,4,5-Tetrachlorpyridin in guter Ausbeute und Reinheit herzustellen. 2,3,4,5-Tetrachlorpyridin stellt ein leicht zugängliches Ausgangsmaterial dar, das beispielsweise nach einem in der US-Patentschrift 3 555 032 beschriebenen Verfahren durch Chlorierung des Hydrochlorids von 2-Chlorpyridin erhalten werden kann, welches seinerseits durch Chlorierung von Pyridin (vgl. US-Patentschriften 2 820 791 und 2 839 534) hergestellt werden kann.

Das erfindungsgemässe Verfahren wird durch die folgenden Beispiele näher erläutert.

Beispiel 1

Eine Suspension von 11,0 g (0,05 Mol) 2,3,4,5-Tetrachlorpyridin und 4,4 g (0,065 Grammatom) Zinkstaub (96,6%ig) in 90 ml Methanphosphonsäuredimethylester wird unter Rühren auf 85 °C erwärmt. Dann wird innerhalb von 35 Minuten eine Lösung von 13,7 g (0,075 Mol) des Tetramethylammoniumsalzes des Methanphosphonsäuremonomethylesters in 31 ml Wasser zugetropft. Nach beendigter Zugabe des Tetramethylammoniumsalzes wird die Temperatur der Mischung noch 1 Stunde bei 85 °C gehalten. Dann wird vom ungelösten Metall abfiltriert und das Filtrat in einer Mischung von 500 ml Eiswasser und 12,5 ml konzentrierter Salzsäure gegossen. Die resultierende weisse Kristallsuspension wird zunächst noch 2 Stunden gerührt und dann filtriert. Der Filterrückstand wird mit 100 ml Wasser gewaschen und über Kaliumhydroxyd getrocknet. Es werden 7,3 g (78,9% d. Th.) 2,3,5-Trichlorpyridin vom Schmelzpunkt 46,5 bis 47,5 °C erhalten, das nach gaschromatographischer Analyse 96,4% 2,3,5-Trichlorpyridin, 0,6% 2,3,4,5-Tetrachlorpyridin und 1,7% unbekannte Produkte enthält.

Das verwendete Tetramethylammoniumsalz des Methanphosphonsäuremethylesters kann wie folgt hergestellt werden:

In einem 1 Liter-Kolben mit aufgesetztem Rückflusskühler werden 540 g (4 Mol) Methanphosphonsäuredimethylester und 219 g (2 Mol) Tetramethylammoniumchlorid unter Rühren auf 130 °C erhitzt, wobei die Reaktion unter Entwicklung von Methylchlorid einsetzt. Die Temperatur wird nun innert 3 Stunden auf 150 °C gesteigert. Danach wird das Reaktionsgemisch noch eine weitere Stunde auf 160 °C erhitzt. Die klare, farblose Lösung wird dann bei 12 Torr zur Trockene eingedampft. Der weisse, kristalline Rückstand wird mit 400 ml Aceton versetzt, 30 Minuten bei 0 °C gerührt und filtriert. Das Produkt wird auf dem Filter mit 500 ml Äther gewaschen und bei 60 °C und 12 Torr über festem Kaliumhydroxyd getrocknet. Es werden 312 g (85% d. Th.) Tetramethylammoniumsalz des Methanphosphonsäure-monomethylesters, F. 172–177 °C (Zers.), in Form von weissen Kristallen erhalten.

Beispiel 2

Nach der in Beispiel 1 beschriebenen Methode werden 11,0 g (0,05 Mol) 2,3,4,5-Tetrachlorpyridin, 4,4 g (0,065 Grammatom) Zinkstaub (95%ig) und 4,0 g (0,075 Mol) Ammoniumchlorid umgesetzt. Es werden 7,1 g (78% d. Th.) 2,3,5-Trichlorpyridin, F. 36–40 °C, erhalten, das nach gaschromatographischer Analyse 79,2% 2,3,5-Trichlorpyridin, 11,8% 2,3,4,5-Tetrachlorpyridin und 6,6% unbekannte Produkte enthält.

Beispiel 3

Nach der in Beispiel 1 beschriebenen Methode werden 11,0 g (0,05 Mol) 2,3,4,5-Tetrachlorpyridin, 4,4 g (0,065 Grammatom) Zinkstaub (96,5%ig) und 24,7 g (0,135 Mol) des Tetramethylammoniumsalzes des Methanphosphonsäuremonomethylesters in 90 ml Trimethylphosphat umgesetzt. Es werden 7,1 g (78% d. Th.) 2,3,5-Trichlorpyridin, 47–47,5 °C erhalten, das nach gaschromatographischer Analyse 97,6% 2,3,5-Trichlorpyridin, 0,4% 2,3,4,5-Tetrachlorpyridin und 1,4% unbekannte Produkte enthält.

Beispiel 4

Nach der in Beispiel 1 beschriebenen Methode werden 11,0 g (0,05 Mol) 2,3,4,5-Tetrachlorpyridin, 4,4 g (0,065 Grammatom) Zinkstaub (96%ig) und 9,5 g (0,075 Mol) des Ammoniumsalzes des Methanphosphonsäuremonomethylesters umgesetzt. Es werden 7,0 g (76% d. Th.) 2,3,5-Trichlorpyridin, F. 37–40 °C erhalten, das nach gaschromatographischer Analyse 78,8% 2,3,5-Trichlorpyridin, 2,5% 2,3,4,5-Tetrachlorpyridin und 0,61% unbekannte Produkte enthält.

Das Ammoniumsalz des Methanphosphonsäure-monomethylesters kann wie folgt hergestellt werden:

In einem 1 Liter-Rundkolben mit aufgesetztem Rückflusskühler werden 540 g (4 Mol) Methanphosphonsäuredimethylester und 107 g (2 Mol) Ammoniumchlorid unter Rühren auf 110 °C erhitzt, wobei die Reaktion unter Entwicklung von Methylchlorid einsetzt. Die Temperatur wird dann innert 20 Minuten auf 138 °C und anschliessend weitere 20 Minuten auf 151 °C erhöht. Danach wird die klare, farblose Lösung bei 12 Torr zur Trockene eingedampft. Als Rückstand wird ein gelbes Öl erhalten, welches nach Zugabe von 500 ml Aceton 24 Stunden gerührt wird, wobei Kristallisation eintritt. Die Kristallsuspension wird auf 0 °C gekühlt, filtriert und anschliessend auf dem Filter unter Ausschluss von Feuchtigkeit mit 150 ml Äther gewaschen. Nach dem Trocknen bei 50 °C und 12 Torr erhält man 118,6 g (46,7% d. Th.) des Ammoniumsalzes des Methanphosphonsäuremonomethylesters, F. 96–103 °C, in Form von stark hygroskopischen Kristallen, die beim Stehen an der Luft bald zerfliessen.

**Patentansprüche**

1. Verfahren zur Herstellung von 2,3,5-Trichlorpyridin, dadurch gekennzeichnet, dass man 2,3,4,5-Tetrachlorpyridin in einem Alkanphosphonsäuredialkylester mit je 1–4 Kohlenstoffatomen in den Alkylgruppen oder in einem Phosphorsäuretrialkylester mit je 1–4 Kohlenstoffatomen in den Alkylgruppen als Lösungsmittel bei 60–120 °C in Gegenwart von 1,4–2,8 Mol pro Mol 2,3,4,5-Tetrachlorpyridin eines Ammoniumsalzes einer anorganischen oder organischen Säure mit 1,2–1,6 Grammatom Zink pro Mol 2,3,4,5-Tetrachlorpyridin umsetzt.

2. Verfahren nach Anspruch 1, dadurch gekennzeichnet, dass man als Alkanphosphonsäuredialkylester Methanphosphonsäuredimethylester oder Äthanphosphonsäurediäthylester verwendet.

3. Verfahren nach Anspruch 1, dadurch gekennzeichnet, dass man als Phosphorsäuretrialkylester Phosphorsäuretrimethylester oder Phosphorsäuretriäthylester verwendet.

4. Verfahren nach Anspruch 1, dadurch gekennzeichnet, dass man die Umsetzung bei 85–90 °C durchführt.

5. Verfahren nach Anspruch 1, dadurch gekennzeichnet, dass man ein Ammoniumsalz der Formel

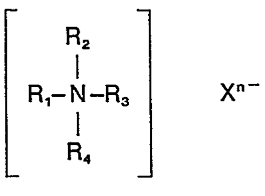

,

in welcher $R_1$, $R_2$, $R_3$ und $R_4$ gleich oder verschieden sein können und je Wasserstoff, Alkyl mit 1 bis 4 Kohlenstoffatomen oder Phenyl, das gegebenenfalls durch Halogen, Alkylgruppen mit 1 bis 4 Kohlenstoffatomen oder Alkoxygruppen mit 1 bis 4 Kohlenstoffatomen substituiert sein kann, bedeuten, X für ein Anion aus der Gruppe Chlorid, Bromid, Sulfat, Hydrogensulfat, Phosphat, Hydrogenphosphat, Dihydrogenphosphat, Carbonat, Hydrogencarbonat, Acetat, Propionat, Butyrat, Isobutyrat, Oxalat, Benzoat, Alkanphosphonat mit 1 bis 4 Kohlenstoffatomen in der Alkylgruppe und Alkan- bzw. Benzolsulfonat mit 1 bis 4 Kohlenstoffatomen in der Alkylgruppe steht und n 1 bis 3 bedeutet und der Zahl der negativen Ladungen des jeweiligen Anions X entspricht, verwendet.

6. Verfahren nach Anspruch 1, dadurch gekennzeichnet, dass man ein Ammoniumsalz des Methansulfonsäuremonomethylesters der Formel

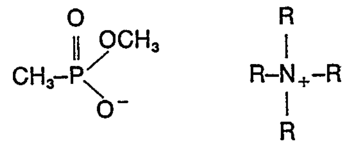

,

in welcher R Wasserstoff oder Methyl bedeutet, verwendet.

7. Verfahren nach Anspruch 1, dadurch gekennzeichnet, dass man als Ammoniumsalz Ammoniumchlorid, Ammoniumsulfat, Ammoniumcarbonat, das Ammoniumsalz des Methanphosphonsäuremonomethylesters oder das Tetramethylammoniumsalz des Methanphosphonsäuremonomethylesters verwendet.

8. Verfahren nach Anspruch 1, dadurch gekennzeichnet, dass man als Ammoniumsalz das Ammoniumsalz des Methanphosphonsäuremonomethylesters oder das Tetramethylammoniumsalz des Methanphosphonsäuremonomethylesters verwendet.

9. Verfahren nach Anspruch 1, dadurch gekennzeichnet, dass man bei Verwendung von Methanphosphonsäuredialkylester als Lösungsmittel 1,6 Mol Ammoniumsalz pro Mol 2,3,4,5-Tetrachlorpyridin einsetzt.

10. Verfahren nach Anspruch 1, dadurch gekennzeichnet, dass man bei Verwendung von Äthanphosphonsäuredialkylester oder Trialkylphosphat als Lösungsmittel 2,6 bis 2,7 Mol Ammoniumsalz pro Mol 2,3,4,5-Tetrachlorpyridin einsetzt.

11. Verfahren nach Anspruch 1, dadurch gekennzeichnet, dass man 1,2–1,3 Grammatom Zink pro Mol 2,3,4,5-Tetrachlorpyridin einsetzt.

12. Ammoniumsalze des Methanphosphonsäuremonomethylesters der Formel

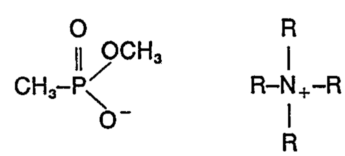

,

in welcher R Wasserstoff oder Methyl bedeutet, wobei höchstens 3 Reste R Methyl bedeuten.

13. Verfahren zur Herstellung von Ammoniumsalzen des Methanphosphonsäuremonomethylesters der Formel

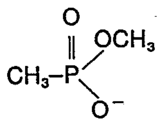 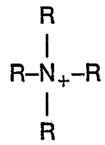 ,

in welcher R Wasserstoff oder Methyl bedeutet, dadurch gekennzeichnet, dass man Ammoniumchlorid oder Tetramethylammoniumchlorid und Methanphosphonsäuredimethylester auf 140–160 °C erhitzt.

14. Verfahren nach Anspruch 13, dadurch gekennzeichnet, dass man die Umsetzung von Ammoniumchlorid oder Tetramethylammoniumchlorid mit Methanphosphonsäuredimethylester in überschüssigem Methanphosphonsäuredimethylester als Lösungsmittel durchführt.

**Claims**

1. A process for producing 2,3,5-trichloropyridine, which process comprises reacting 2,3,4,5-tetrachloropyridine, in an alkanephosphonic acid dialkyl ester having 1 to 4 carbon atoms in each of the alkyl groups or in a phosphoric acid trialkyl ester having 1 to 4 carbon atoms in each of the alkyl groups as the solvent, at 60–120 °C, in the presence of 1.4 to 2.8 mols, per mol of 2,3,4,5-tetrachloropyridine, of an ammonium salt of an inorganic or organic acid, with 1.2 to 1.6 gram atoms of zinc per mol of 2,3,4,5-tetrachloropyridine.

2. A process according to Claim 1, wherein the alkanephosphonic acid dialkyl ester used is methanephosphonic acid dimethyl ester or ethanephosphonic acid diethyl ester.

3. A process according to Claim 1, wherein the phosphoric acid trialkyl ester used is phosphoric acid trimethyl ester or phosphoric acid triethyl ester.

4. A process according to Claim 1, wherein the reaction is performed at 85–90 °C.

5. A process according to Claim 1, wherein there is used an ammonium salt of the formula

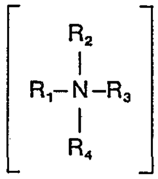 $X^{n-}$ ,

in which $R_1$, $R_2$, $R_3$ and $R_4$ can be identical or different and are each hydrogen, alkyl having 1 to 4 carbon atoms, or phenyl which can be substituted by halogen, by alkyl groups having 1 to 4 carbon atoms, or by alkoxy groups having 1 to 4 carbon atoms, X is an anion from the group: chloride, bromide, sulfate, hydrogen sulfate, phosphate, hydrogen phosphate, dihydrogen phosphate, carbonate, hydrogen carbonate, acetate, propionate, butyrate, isobutyrate, oxalate, benzoate, alkanephosphonate having 1 to 4 carbon atoms in the alkyl group and alkane- or benzenesulfonate having 1 to 4 carbon atoms in the alkyl group, and n is 1 to 3 and corresponds to the number of negative charges of the respective anion X.

6. A process according to Claim 1, wherein there is used an ammonium salt of the methanesulfonic acid monomethyl ester of the formula

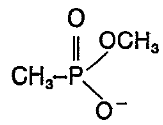 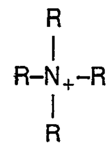

in which R is hydrogen or methyl.

7. A process according to Claim 1, wherein the ammonium salt used is ammonium chloride, ammonium sulfate, ammonium carbonate, the ammonium salt of methanephosphonic acid monomethyl ester or the tetramethylammonium salt of methanephosphonic acid monomethyl ester.

8. A process according to Claim 1, wherein the ammonium salt used is the ammonium salt of methanephosphonic acid monomethyl ester or the tetramethylammonium salt of methanephosphonic acid monomethyl ester.

9. A process according to Claim 1, wherein, with the use of methanephosphonic acid dialkyl ester as solvent, there are used 1.6 mols of ammonium salt per mol of 2,3,4,5-tetrachloropyridine.

10. A process according to Claim 1, wherein, with the use of ethanephosphonic acid dialkyl ester or trialkyl phosphate as solvent, there are used 2.6 to 2.7 mols of ammonium salt per mol of 2,3,4,5-tetrachloropyridine.

11. A process according to Claim 1, wherein 1.2–1.3 gram atoms of zinc are used per mol of 2,3,4,5-tetrachloropyridine.

12. An ammonium salt of methanephosphonic acid monomethyl ester of the formula

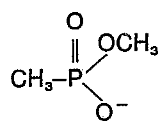 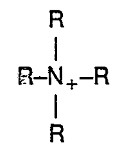

in which R is hydrogen or methyl, at most 3 radicals R being methyl.

13. A process for producing ammonium salts of the methanephosphonic acid monomethyl ester of the formula

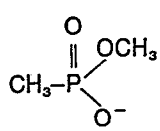 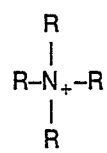

in which R is hydrogen or methyl, wherein ammonium chloride or tetramethylammonium chloride and methanephosphonic acid dimethyl ester are heated to 140–160 °C.

14. A process according to Claim 13, wherein the reaction of ammonium chloride or tetramethylammonium chloride with methanephosphonic acid dimethyl ester is performed in excess methanephosphonic acid dimethyl ester as solvent.

## Revendications

1. Procédé de préparation de 2,3,5-trichloropyridine, caractérisé en ce qu'on fait réagir de la 2,3,4,5-tétrachloropyridine dans un dialcoylester d'acide alcanephosphonique avec à chaque fois de 1 à 4 atomes de carbone dans les groupes alcoyle ou dans un trialcoylester d'acide phosphorique avec à chaque fois de 1 à 4 atomes de carbone dans les groupes alcoyle comme solvant à 60–120 °C en présence de 1,4–2,8 mole par mole de 2,3,4,5-tétrachloropyridine d'un sel d'ammonium d'un acide inorganique ou organique avec 1,2–1,6 atome-gramme de zinc par mole de 2,3,4,5-tétrachloropyridine.

2. Procédé selon la revendication 1, caractérisé en ce qu'on utilise comme dialcoylester d'acide alcanephosphonique le diméthylester de l'acide méthanephosphonique ou le diéthylester de l'acide éthanephosphonique.

3. Procédé selon la revendication 1, caractérisé en ce qu'on utilise comme trialcoylester de l'acide phosphorique le triméthylester de l'acide phosphorique ou le triéthylester de l'acide phosphorique.

4. Procédé selon la revendication 1, caractérisé en ce qu'on conduit la réaction à 85–90 °C.

5. Procédé selon la revendication 1, caractérisé en ce qu'on utilise un sel d'ammonium de formule

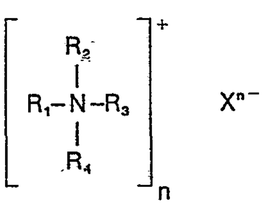

où $R_1$, $R_2$, $R_3$ et $R_4$ peuvent être semblables ou différents et représentent chacun un hydrogène, un alcoyle en $C_1$ à $C_4$ ou un phényle, qui peut être éventuellement substitué par un halogène, des groupes alcoyle en $C_1$ à $C_4$ ou des groupes alcoxy en $C_1$ à $C_4$, X représente un anion du groupe chlorure, bromure, sulfate, sulfate acide, phosphate, phosphate acide, phosphate diacide, carbonate, carbonate acide, acétate, propionate, butyrate, isobutyrate, oxalate, benzoate, alcanephosphonate ayant de 1 à 4 atomes de carbone dans le groupe alcoyle et alcane- ou selon les cas benzènesulfonate ayant de 1 à 4 atomes de carbone dans le groupe alcoyle et n vaut de 1 à 3 et correspond au nombre de charges négatives de l'anion X correspondant.

6. Procédé selon la revendication 1, caractérisé en ce qu'on utilise un sel d'ammonium du monométhylester de l'acide méthanesulfonique de formule

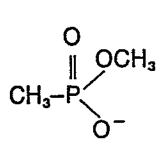

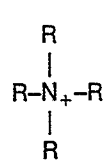

où R représente un hydrogène ou un méthyle.

7. Procédé selon la revendication 1, caractérisé en ce qu'on utilise comme sel d'ammonium le chlorure d'ammonium, le sulfate d'ammonium, le carbonate d'ammonium, le sel d'ammonium du monométhylester de l'acide méthanephosphonique ou le sel de tétraméthylammonium du monométhylester de l'acide méthanephosphonique.

8. Procédé selon la revendication 1, caractérisé en ce qu'on utilise comme sel d'ammonium le sel d'ammonium du monométhylester de l'acide méthanephosphonique ou le sel de tétraméthylammonium du monométhylester de l'acide méthanephosphonique.

9. Procédé selon la revendication 1, caractérisé en ce qu'on utilise, lorsqu'on emploie un dialcoylester d'acide méthanephosphonique comme solvant, 1,6 mole de sel d'ammonium par mole de 2,3,4,5-tétrachloropyridine.

10. Procédé selon la revendication 1, caractérisé en ce qu'on utilise, lorsqu'on emploie un dialcoylester d'acide éthanephosphonique ou du trialcoylphosphate comme solvant, de 2,6 à 2,7 moles de sel d'ammonium par mole de 2,3,4,5-tétrachloropyridine.

11. Procédé selon la revendication 1, caractérisé en ce qu'on utilise 1,2–1,3 atome-grammes de zinc par mole de 2,3,4,5-tétrachloropyridine.

12. Sels d'ammonium du monométhylester de l'acide méthanephosphonique de formule

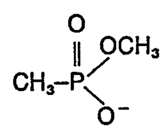

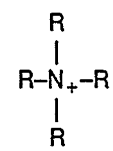

où R représente un hydrogène ou un méthyle, et où au plus trois radicaux R représentent un méthyle.

13. Procédé de préparation de sels d'ammonium du monométhylester de l'acide méthanephosphonique de formule

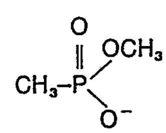

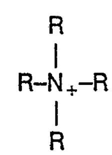

où R représente un hydrogène ou un méthyle, caractérisé en ce qu'on chauffe à 140–160 °C du chlorure d'ammonium ou du chlorure de tétraméthylammonium et du diméthylester de l'acide méthanephosphonique.

14. Procédé selon la revendication 13, caractérisé en ce qu'on conduit la réaction de chlorure d'ammonium ou de chlorure de tétraméthylammonium avec du diméthylester de l'acide méthanephosphonique dans un excès de diméthylester de l'acide méthanephosphonique comme solvant.